# EUROPEAN PATENT SPECIFICATION

(11) **EP 2 535 047 B1**
(45) Date of publication and mention of the grant of the patent: **28.11.2018**
(21) Application number: 12172280.5
(22) Date of filing: 15.06.2012
(51) Int. Cl.: A61K 31/194, A61K 31/7004, A61K 31/7048, A61K 31/733, A61K 45/06, A61K 9/00, A61P 13/02, A61K 33/00, A61K 9/14, A61K 9/16, A23L 33/105, A23L 33/16, A23L 33/21

(54) **Nutritional composition, food supplement comprising said nutritional composition and kit comprising said food supplement**
Ernährungszusammensetzung, nahrungsergänzungsmittel mit besagter ernährungszusammensetzung und kit mit besagtem nahrungsergänzungsmittel
Composition nutritionnelle, complément alimentaire comprenant ladite composition nutritionnelle et kit comprenant ledit complément alimentaire

(30) Priority: 16.06.2011 IT CN20110006
(43) Date of publication of application: 19.12.2012
(73) Proprietor: Alpiflor S.R.L., 12026 Piasco (CN) (IT)
(72) Inventor: Scatolero, Daniele, 12026 Piasco (CN) (IT)
(74) Representative: Robba, Pierpaolo

(56) References cited:
- WO-A1-2004/047810
- WO-A1-2012/041898
- FR-A1- 2 906 109
- US-A1- 2009 175 843
- US-A1- 2011 064 706
- PRINCEPS: "Utimann Integratore Alimentare Rifase", 20111021, [Online] 21 October 2011 (2011-10-21), pages 1-3, XP007920999, Retrieved from the Internet: URL:http://www.princeps.it/db_files/prodot ti/utimann.pdf> [retrieved on 2012-09-07]
- HEAD K A: "Natural approaches to prevention and treatment of infections of the lower urinary tract", ALTERNATIVE MEDICINE REVIEW, THORNE RESEARCH INC., SANDPOINT, US, vol. 13, no. 3, 1 September 2008 (2008-09-01), pages 227-244, XP009148596, ISSN: 1089-5159

## Description

### Technical field

The present invention relates to a nutritional composition.

More particularly, the present invention relates to a nutritional composition that allows yielding benefits to the urinary apparatus in general and, more particularly, fighting against urinary tract infections caused by pathogenic germs including, above all, *Escherichia coli,* but also other germs such as *Staphylococcus aureus, Klebsiella, Pseudomonas, Enterobacter, Proteus, Staphylococcus, Mycoplasma, Chlamydia, Serratia* and *Neisseria spp.*

The present invention also relates to a kit comprising the nutritional composition mentioned above and providing the user with the means for yielding substantial benefits to the urinary apparatus and fighting against urinary tract infections.

### Prior Art

Most of urinary tract infections are caused by Gram-negative bacteria. In particular, *Escherichia coli,* a bacterial strain usually living in colon, is one of the commonest causes of urinary tract infections (about 80% of urinary tract infections).

Yet, many other bacteria - even though less frequently - can sometimes cause an infection (for instance, *Klebsiella, Pseudomonas, Enterobacter, Proteus, Staphylococcus, Mycoplasma, Chlamydia, Serratia and Neisseria spp*). Moreover, also some fungi (such as for instance *Candida* and *Cryptococcus ssp*) and some parasites (*Trychomonas, Schistosoma*) may cause urinary tract infections.

The commonest treatment for such kind of infections is the antibiotic systemic therapy, which however entails a number of risks related to the antibiotic used and the onset of side effects related to the drug (intestinal diseases resulting from the onset of dismicrobisms due to the systemic treatment, abdominal swelling, diarrhoea and so on).

In recent years, it has been discovered that some sugars exhibit a marked affinity for some germs responsible for urinary tract infections and are capable of effectively acting to solve the pathology, yet without having the side effects of the antibiotic therapy.

In particular, products containing D-mannose have been put in commerce and have given good results in the therapy of urinary infections.

D-mannose is a dextrorotatory, hexose aldehyde monosaccharide, extracted from birch or another vegetable or obtained by chemical synthesis or by modification of other sugars.

Generally, in said known products, D-mannose is associated with extracts of cranberry (*Vaccinium Macrocarpum*).

In association with or in the alternative to said components, food supplements containing prebiotics, probiotics or both ("symbiotics") are used in order to reduce the intestinal flora responsible for the infections and to reduce the effects of the antibiotic therapy.

Examples of nutritional compositions containing D-mannose in association with cranberry extracts and possibly with prebiotics and/or probiotics are known for instance from documents US 2011/0064706, US 2009/0175843 and FR 2,906,109.

Moreover, in HEAD K.A.: "Natural approaches to prevention and treatment of infections of the lower urinary tract", ALTERNATIVE MEDICINE REVIEW, THORNE RESEARCH INC., SANDPOINT, US, vol. 13, no. 3, 1 September 2008 (2008-09-01), pages 227-244, an overview of natural compositions and botanicals suitable for treating and preventing urinary tract infections is given. Large relevance is given, in particular, to the use of cranberry (*Vaccinium macrocarpon*) and other similar berries (billberry, blueberry, bearberry). Other natural substances that could have beneficial effects include vitamin C, vitamin A, citrate salts and D-mannose.

Yet, the Applicant has realised that, in general, in prior art nutritional compositions containing D-mannose, the effectiveness of D-mannose in treating urinary tract infections is in practice limited.

Such products are only partly effective in treating urinary tract infections and, in particular, they do not effectively contribute to reducing the risk of reinfection, related for instance to anatomic and/or physiologic situations, or resulting from functional malformations, pathologies deriving from vesical emptying and/or pathologies of the upper urinary tract.

It is an object of the present invention to provide a nutritional composition that allows optimising the effectiveness of D-mannose in treating urinary tract infections and that, thanks to the coordinate action of all ingredients, allows not only treating urinary tract infections already in progress, but also reducing the risk of urinary tract infection and reinfection.

It is another object of the present invention to provide a kit comprising the nutritional composition mentioned above, which kit, in the whole, provides the user with the most suitable tools for treating urinary tract infections already in progress and preventing the risk of urinary tract infection and reinfection.

The above and other objects are achieved by means of a nutritional composition and a kit as claimed in the appended claims.

### Description of the invention

As stated above, nutritional compositions for treating urinary tract infections generally contain D-mannose in association with cranberry extracts.

The Applicant has surprisingly realised that, contrary to the universal belief, cranberry extract does not have a positive effect on the effectiveness of D-mannose.

On the contrary, said cranberry extract negatively affects the effectiveness of D-mannose in treating urinary tract infections.

Such a surprising result is to be found in that the presence of cranberry leads to an acidification of the urines and to a drastic pH reduction, bringing the pH itself into a range of values inside which the effectiveness of D-mannose is limited.

The presence, in many of the prior art compositions, of further additives leading to an even more marked pH reduction - first of all citric acid, often used in the prior art in association with D-mannose - entails a further reduction of the effectiveness of D-mannose itself.

In the nutritional composition according to the invention, thanks to the association of D-mannose with a pH stabiliser, the effectiveness of D-mannose is optimised.

In the present context, "pH stabiliser" means an additive capable of bringing pH to and maintaining pH in a range of values from about 6.0 to about 6.5.

Based on experimental tests, the Applicant has noticed that, at such pH values, the effectiveness of D-mannose is optimised.

According to a preferred embodiment of the invention, D-mannose is D-mannose obtained by extraction from birch.

Moreover, according to the invention, said pH stabiliser comprises
one or more citrates of alkaline metals and/or alkaline-earth metals, selected from magnesium citrate and potassium citrate.

In this respect, it will be apparent to the skilled in the art how the use of citrates of alkaline and/or alkaline-earth metals is very different from the use for instance of citric acid, often used as an additive in the prior art.

Indeed, the presence of citric acid leads to an acidification of the urines and this, as realised by the Applicant, has a worsening effect on the effectiveness of D-mannose.

On the contrary, the addition of citrates leads to an alkalinisation of the urines.

Alkalinisation of the urines is a process of voluntary modification of the pH of the renal filtrate, and hence of the haematic filtrate. It is essential to attempt to alkalinise the urines in case of onset for instance of metabolic acidosis, which is an unfavourable condition for metabolic homeostasis induced by an excess presence of acid molecules. By the alkalinisation of the urines, it is possible to obtain pH values of the urine filtrate in the range 5.0 to 8.5 (as known, neutral pH is 7.0). Advantageously, the citrates, besides effectively alkalinising the urines, are directly eliminated, thereby preventing drug crystallisation-precipitation and consequently preventing renal calculosis.

The nutritional composition according to the invention also includes one or more prebiotics.

Prebiotics operate at intestinal level to promote the competitive growth of intestinal saprophyte microorganisms and, consequently, they assist in restoring the saprophytic flora to the expenses of the potentially pathogenic flora and promote, through the enhancement of intestinal peristalsis, the expulsion of potential pathogens responsible for reinfection.

According to a preferred embodiment of the invention, the prebiotic being used is inulin.

The nutritional composition also includes one or more bioflavonoids.

Actually bioflavonoids contribute to reducing inflammation and hence they reduce pathologic modifications of the epithelium of the urinary tract, reduce tissue exudate and create again the optimum ground for the elimination of pathogenic germs.

It will be clearly apparent to the skilled in the art that the choice of the bioflavonoids to be used first of all must not be in contrast with the action of the pH stabiliser. In other words, the bioflavonoids being used must have a negligible effect on the pH.

Thus, it is self evident in this respect that cranberry extracts and the like are to be avoided.

On the contrary, bioflavonoids like diosmin and hesperidin are preferably used.

The nutritional composition according to the invention is preferably administered to the users as a food supplement.

The nutritional composition according to the invention as defined above promotes the growth of saprophytic flora in intestine, in order to reduce the presence of pathogenic germs responsible for reinfections, and effectively contributes to eliminating germs present in the urinary tract and to reducing the symptoms related to the infection by quickly restoring homeostasis of the concerned organs.

Advantageously, the nutritional composition according to the invention allows the consumer suffering from urinary tract infections to recover more rapidly the state of physiological well-being both at the level of the urinary apparatus and at the level of the gastroenteric apparatus by means of a single product.

The focal feature of the invention is that all present ingredients act synergistically together in adjuvant manner, so that the activities of the individual ingredients are made more powerful, thereby resulting in a more active product, whose activity even exceeds the sum the activities of the individual ingredients.

Such a synergistic result is substantially achieved thanks to the presence of the pH stabiliser maintaining the pH in a range from about 6.0 to about 6.5.

Actually, the association with the citrates allows maintaining the optimum pH for avoiding chemical reactions consequent to acidic pH variations, which are on the contrary induced by the additives used in the prior art, such as cranberry extracts and/or citric acid, and which would reduce the effectiveness of D-mannose.

By means of laboratory tests and tests performed on a sample of patients suffering from urinary tract infection, the Applicant has surprisingly noticed that, thanks to the administration of the nutritional composition according to the invention, it is possible to significantly improve the beneficial action of D-mannose on the germs responsible for the infection and to quickly reduce the related symptoms down to their disappearance. Advantageously, it came out that D-mannose wholly performs its action on the germs present in the vesical tract by absorbing them and favouring their removal from the urinary tract, while at the same time inulin promotes a selective growth of bifido-bacteria to the expenses of entero-pathogenic bacteria, which are eliminated with the faeces.

Moreover, the presence of citrates contributes to maintaining the urinary pH at a level unfavourable for the growth of germs in the urinary tract and hence to reducing the risk of reinfection, especially in women.

Lastly, the presence of flavonoids, thanks to their astringent and anti-inflammatory activity, quickly reduces hematuria and inflammation down to their disappearance.

Hence, the nutritional composition according to the invention allows bringing into the urinary tract and at the same time into the intestine all necessary elements for reducing urinary tract infection and for reducing the risk of reinfection in predisposed subjects, whereby the objects set above are achieved.

The present invention also relates to a kit comprising the nutritional composition disclosed above and a further nutritional composition containing D-mannose.

Actually, the Applicant has realised that, in order to obtain effective results, it is necessary to introduce a sufficient quantity of D-mannose into the organism, which quantity is to be taken in small doses and frequently.

Indeed, D-mannose is practically not absorbed by the organism and is eliminated in the urines. Thus, it is to be taken at short time intervals.

The invention provides therefore a kit comprising:
- a first food supplement comprising the nutritional composition according to the invention; and
- a second food supplement comprising a second nutritional composition containing D-mannose in combination with bioflavonoids.

According to a preferred embodiment of the invention, said kit comprises:
1) a first food supplement comprising the composition according to the invention, in which in particular the pH stabiliser is a citrate and in which said composition also includes a prebiotic, preferably inulin, said first food supplement being prepared in the form of powder or granules; and
2) a second food supplement comprising a second composition in which D-mannose and the bioflavonoids, in particular diosmin and/or hesperidin, are associated together, said second food supplement being prepared in the form of pills, tablets or capsules.

D-mannose subdivided in this manner and hence administered in different times has an effectiveness exceeding that of the hypothetical administration of all ingredients together taken all at once, and is capable of yielding even more significant benefits both to the intestine and to the urinary tract.

By way of example, the kit according to the invention could comprise:
1) First food supplement: powder or granule mixture with the following composition:

| *Ingredient* | *grams* |
|---|---|
| D-Mannose | 7.50000 |
| Inulin | 39.4700 |
| Magnesium citrate | 1.50000 |
| Micronized silica (Aerosil) | 0.03000 |
| Potassium citrate | 1.50000 |

2) Second food supplement: tablets with the following composition:

| *Ingredient* | *grams* |
|---|---|
| D-Mannose | 0.50000 |
| Diosmin | 0.01000 |
| Hesperidin | 0.08000 |
| Magnesium stearate | 0.01000 |

Preferably, the use of the kit comprises administering the powder or granule mixture containing the nutritional composition according to the invention in the morning, before breakfast, and in the evening, before going to bed (about 5 g of powder or granules for each administration), and administering one tablet or capsule containing the second nutritional composition at the middle of the morning, after lunch and at the middle of the afternoon.

More generally, according to the invention, in order to obtain a proper treatment of urinary tract infections, a daily administration is envisaged that, in the whole, comprises:
D-Mannose 0.05 to 15 g
Inulin 0.05 to 100 g
Citrates 0.05 to 25 g
Bioflavonoids 0.05 to 2,000 mg
and preferably:
D-Mannose 0.5 to 5 g
Inulin 0.5 to 10 g
Citrates 0.5 to 10 g
Bioflavonoids 0.05 to 1 g.

By way of example only, the food supplements in the kit according to the invention can be prepared in the way described hereinafter.

The ingredients included in the nutritional composition according to the invention are separately weighed in suitable containers and then they are transferred into a suitable mixer and mixed for at least 15 min. At the end of the mixing step, the quality controls provided by the quality control procedure are performed. Subsequently, the mixture prepared in this manner is packaged into containers of plastics suitable for food. Doses of 50 g of the mixture for each container are to be provided. The powder mixture obtained in this manner has the following characteristics:

| mixture uniformity | |
|---|---|
| granulometry | 90% below 150 micrometres |
| humidity | < 6% |
| TAMC | < 1000 UFC/g |
| TYMC | < 100 UFC/g |
| Salmonella | absent in 10 g |
| Coliforms | absent |
| Staphylococcus aureus | absent |
| Listeria | absent |

The ingredients included in the second nutritional composition are separately weighed. Then, D-mannose, diosmin and hesperidin are transferred into a suitable mixer and mixed for 15 min. Subsequently, magnesium stearate is added and mixing is carried out for further 5 min. By using a compressing or capsule filling machine, the tablets or the capsules are prepared. Once the compression is over, the primary packaging of the tablets/capsules into PVC/PVDC blisters is performed.

The tablets/capsules obtained in this manner have the following characteristics:

| | |
|---|---|
| mass uniformity | ± 3% |
| friability loss | < 2% |
| maximum breakage stress | 98 Newtons ± 3% |
| humidity | < 10% |
| breakup | max 20' |
| size | diameter 11 mm |
| thickness | 5.6 mm ± 3% |
| TAMC | < 1000 UFC/g |
| TYMC | < 100 UFC/g |
| Salmonella | absent in 10 g |
| Coliforms | absent |
| Staphylococcus aureus | absent |
| Listeria | absent |

## Claims

1. A nutritional composition for use in the treatment and prevention of urinary tract infections, the composition comprising D-mannose in any non-null dosage, further including at least one prebiotic in any non-null dosage and further including at least one bioflavonoid in any non-null dosage, **characterised in that** it comprises a pH stabiliser in any non-null dosage, said pH stabiliser comprising magnesium citrate and/or potassium citrate.

2. The nutritional composition for use as claimed in claim 1, wherein said at least one prebiotic is inulin.

3. The nutritional composition for use claimed in claim 1, wherein said at least one bioflavonoid is selected from the group comprising diosmin and hesperidin.

4. A kit of food supplements, **characterised in that** it comprises:
- a first food supplement comprising a first nutritional composition comprising D-mannose in any non-null dosage, further including at least one prebiotic in any non-null dosage and further including a pH stabiliser in any non-null dosage, said pH stabiliser comprising magnesium citrate and/or potassium citrate; and
- a second food supplement comprising a second nutritional composition containing D-mannose in any non-null dosage and at least one bioflavonoid.

5. The kit as claimed in claim 4, wherein said at least one prebiotic is inulin.

6. The kit as claimed in claim 4 or 5, wherein said at least one bioflavonoid is selected from the group comprising diosmin and hesperidin.

7. The kit as claimed in claim 4 or 5 or 6, wherein said first food supplement is prepared in the form of powder or granules and said second food supplement is prepared in the form of pills, capsules or tablets.

8. The kit as claimed in claim 4, wherein said first food supplement is prepared in the form of powder or granules and said nutritional composition in said first food supplement comprises:
- D-Mannose 7.5 g
- Magnesium citrate 1.5 g
- Potassium citrate 1.5 g
- Inulin 39.47 g
- Micronized silica 0.03 g and wherein said second food supplement is prepared in the form of pills, capsules or tablets and said second nutritional composition in said second food supplement comprises:
- D-Mannose 0.5 g
- Diosmin 0.01 g
- Hesperidin 0.08 g
- Magnesium stearate 0.01 g.

## Patentansprüche

1. Nahrungszusammensetzung zur Verwendung in der Behandlung und Prävention von Infektionen der Harnwege, wobei die Zusammensetzung D-Mannose in einer von Null verschiedenen Dosierung und zusätzlich wenigstens ein Präbiotikum in einer von Null verschiedenen Dosierung sowie wenigstens ein Bioflavonoid in einer von Null verschiedenen Dosierung enthält, **dadurch gekennzeichnet, dass** sie einen pH-Stabilisator in einer von Null verschiedenen Dosierung enthält, wobei der pH-Stabilisator Magnesiumzitrat und oder Kaliumzitrat enthält.

2. Nahrungszusammensetzung zur Verwendung wie in Anspruch 1 beansprucht, wobei das wenigstens eine Präbiotikum Inulin ist.

3. Nahrungszusammensetzung zur Verwendung wie in Anspruch 1 beansprucht, wobei das wenigstens eine Bioflavonoid aus der Gruppe ausgewählt ist, die Diosmin und Hesperidin enthält.

4. Satz von Nahrungsergänzungsmitteln, **dadurch gekennzeichnet**, das er enthält:
- eine erste Nahrungsergänzung mit einer ersten Nahrungszusammensetzung, die D-Mannose in eine von Null verschiedenen Dosierung und zusätzlich wenigstens ein Präbiotikum in einer von Null verschiedenen Dosierung und zusätzlich einen pH-Stabilisator in einer von Null verschiedenen Dosierung enthält, wobei der pH-Stabilisator Magnesiumzitrat und / oder Kaliumzitrat enthält, und
- ein zweites Nahrungsergänzungsmittel mit einer zweiten Nahrungszusammensetzung, die D-Mannose in einer von Null verschiedenen Dosierung und wenigstens ein Bioflavonoid enthält.

5. Satz nach Anspruch 4, wobei das wenigstens eine Präbiotikum Inulin ist.

6. Satz nach Anspruch 4 oder 5, wobei das wenigstens eine Bioflavonoid aus der Gruppe ausgewählt ist, die Diosmin und Hesperidin enthält.

7. Satz nach einem der Ansprüche 4 oder 5 oder 6, wobei das erste Nahrungsergänzungsmittel in Form von Pulver oder Granulat zubereitet ist und das zweite Nahrungsergänzungsmittel in Form von Pillen, Kapseln oder Tabletten zubereitet ist.

8. Satz nach Anspruch 4, wobei das erste Nahrungsergänzungsmittel in Form von Pulver oder Granulat und die Nahrungszusammensetzung in dem ersten Nahrungsergänzungsmittel enthält:
- D-Mannose 7,5 g
- Magnesiumzitrat 1,5 g
- Kaliumzitrat 1,5 g
- Inulin 39,47 g
- Mikronisierte Kieselerde 0,03 g
und wobei das zweite Nahrungsergänzungsmittel in Form von Pillen, Kapseln oder Tabletten zubereitet ist und die zweite Nahrungszusammensetzung in dem zweiten Nahrungsergänzungsmittel enthält:
- D-Mannose 0,5 g
- Diosmin 0,01 g
- Hesperidin 0,08 g
- Magnesiumstearat 0,01 g

## Revendications

1. Composition nutritionnelle pour une utilisation dans le traitement et la prévention d'infections du tractus urinaire, la composition comprenant du D-mannose à tout dosage non nul, comprenant en outre au moins un prébiotique à tout dosage non nul, et comprenant en outre au moins un bioflavonoïde à tout dosage non nul, **caractérisée par le fait qu'**elle comprend un stabilisateur de pH à tout dosage non nul, ledit stabilisateur de pH comprenant du citrate de magnésium et/ou du citrate de potassium.

2. Composition nutritionnelle pour une utilisation selon la revendication 1, dans laquelle ledit au moins un prébiotique est de l'inuline.

3. Composition nutritionnelle pour une utilisation selon la revendication 1, dans laquelle ledit au moins un bioflavonoïde est choisi parmi le groupe comprenant la diosmine et l'hespéridine.

4. Kit de compléments alimentaires, **caractérisé par le fait qu'**il comprend :
- un premier complément alimentaire comprenant une première composition nutritionnelle comprenant du D-mannose à tout dosage non nul, comprenant en outre au moins un prébiotique à tout dosage non nul, et comprenant en outre au moins un stabilisateur de pH à tout dosage non nul, ledit stabilisateur de pH comprenant du citrate de magnésium et/ou du citrate de potassium ; et
- un second complément alimentaire comprenant une seconde composition nutritionnelle contenant du D-mannose à tout dosage non nul et au moins un bioflavonoïde.

5. Kit selon la revendication 4, dans lequel l'au moins un prébiotique est de l'inuline.

6. Kit selon la revendication 4 ou 5, dans lequel ledit au moins un bioflavonoïde est choisi parmi le groupe comprenant la diosmine et l'hespéridine.

7. Kit selon la revendication 4 ou 5 ou 6, dans lequel ledit premier complément alimentaire est préparé sous la forme de poudre ou de granulés, et ledit second complément alimentaire est préparé sous la forme de pilules, de capsules ou de comprimés.

8. Kit selon la revendication 4, dans lequel ledit premier complément alimentaire est préparé sous la forme de poudre ou de granulés, et ladite composition nutritionnelle dans ledit premier complément alimentaire comprend :
- D-mannose 7,5 g
- Citrate de magnésium 1,5 g
- Citrate de potassium 1,5 g
- Inuline 39,47 g
- Silice micronisée 0,03 g
et ledit second complément alimentaire est préparé sous la forme de pilules, de capsules ou de comprimés, et ladite seconde composition nutritionnelle dans ledit second complément alimentaire comprend :
- D-mannose 0,5 g
- Diosmine 0,01 g
- Hespéridine 0,08 g
- Stéarate de magnésium 0,01 g.
